# EUROPEAN PATENT SPECIFICATION

(11) **EP 3 583 195 B1**
(45) Date of publication and mention of the grant of the patent: **20.12.2023**
(21) Application number: 18706563.6
(22) Date of filing: 15.02.2018
(51) Int. Cl.: C10M 105/18, C10M 177/00, C07C 41/06, C10N 20/00, C10N 20/02, C10N 30/00, C10N 70/00

(54) **ETHERIFICATION PROCESS, PROCESS FOR PREPARING A LUBRICANT COMPOSITION AND PROCESS FOR LUBRICATING A SURFACE**
VERETHERUNGSVERFAHREN, VERFAHREN ZUR HERSTELLUNG EINER SCHMIERSTOFFZUSAMMENSETZUNG UND VERFAHREN ZUR SCHMIERUNG EINER OBERFLÄCHE
PROCÉDÉ D'ÉTHÉRIFICATION, PROCÉDÉ DE PRÉPARATION D'UNE COMPOSITON LUBRIFIANTE ET PROCÉDÉ DE LUBRIFICATION D'UNE SURFACE

(30) Priority: 15.02.2017 GB 201702456
(43) Date of publication of application: 25.12.2019
(73) Proprietor: BP P.L.C., London SW1Y 4PD (GB)
(72) Inventor: ARMITAGE, Gareth, London SW1Y 4PD (GB); DEELEY, Jon, London SW1Y 4PD (GB)
(74) Representative: Hamer, Christopher K.
(86) International application number: PCT/GB2018/050407
(87) International publication number: WO 2018/150185

(56) References cited:
- WO-A1-2014/207235
- WO-A1-2016/203310
- US-A- 3 281 475
- US-A1- 2013 109 604
- US-B1- 9 193 653

## Description

The present invention relates to a process for preparing ethers, particularly unsymmetrical ethers, suitable for use as base stocks for lubricant compositions. In particular, the process involves the reaction of an alcohol and alkene in the presence of an acidic ion-exchange resin etherification catalyst and/or an acidic aluminosilicate etherification catalyst, wherein the alkene is present in molar excess relative to the alcohol.

### Background

Lubricating compositions generally comprise a base oil of lubricating viscosity together with one or more additives to deliver properties including for example, reduced friction and wear, improved viscosity index, improved dispersancy, detergency, and resistance to oxidation and corrosion. A lubricant base oil may comprise one or more lubricating base stocks.

Lubricant base stocks used in automotive engine lubricants are generally obtained from petrochemical sources, for example they may be obtained as the higher boiling fractions isolated during the refining of crude oil or as the products of chemical reactions of feedstocks from petrochemical sources. Lubricant base stocks can also be made from Fischer-Tropsch wax.

Lubricant base stocks may be classified as Group I, II, III, IV and V base stocks according to API standard 1509, "ENGINE OIL LICENSING AND CERTIFICATION SYSTEM", 17th Edition, Annex E (October 2013 with Errata March 2015), as set out in Table 1.

**Table 1**

| Group | Saturated hydrocarbon content (% by weight) ASTM D2007 | | Sulphur content (% by weight) ASTM D2622, D4294, D4927, D3120 orD1552 | | Viscosity Index ASTM D2270 |
|---|---|---|---|---|---|
| I | < 90 | and/or | > 0.03 | and | ≥ 80 and < 120 |
| II | ≥ 90 | and | ≤ 0.03 | and | ≥ 80 and < 120 |
| III | ≥ 90 | and | ≤ 0.03 | and | ≥ 120 |
| IV | Polyalphaolefins | | | | |
| V | all base stocks not in Groups I, II, III or IV | | | | |

Group I base stocks are typically manufactured by known processes including, for example, solvent extraction and solvent dewaxing, or solvent extraction and catalytic dewaxing. Group II and Group III base stocks are typically manufactured by known processes including, for example, catalytic hydrogenation and/or catalytic hydrocracking, and catalytic hydroisomerisation. Group IV base stocks include for example, hydrogenated oligomers of alpha olefins.

A combination of properties is desirable in a base stock. In some instances, for example in passenger car engine oils, it may be desirable for a base stock to confer a low viscosity profile on the lubricant composition, since this leads to improved fuel economy. In particular, it is desirable for base stocks to have a low kinematic viscosity as well as good low-temperature viscosity characteristics, for example a low pour point or low viscosity as measured using a mini-rotary viscometer (MRV). However, the general trend is for an improvement in the viscosity profile (*i.e.* a reduction in viscosity parameters) of a base oil to be accompanied by an undesirable increase in volatility. Desirable base stocks are therefore those having low volatility for a given viscosity profile, but which are also suitable for use, for example, in a lubricating composition for an internal combustion engine. Ether base stocks have been found by the inventors to be particularly useful in conferring desirable properties on lubricant compositions.

Known processes for preparing ethers include the reaction of an alcohol with an alkyl group having a suitable leaving group, such as a halogen (for example bromine, chlorine or iodine) or a sulfonate ester (for example mesylate or tosylate), in the presence of a base (for example potassium hydroxide or potassium tert-butoxide) and a catalyst (for example Starks' catalyst: *N*-Methyl-*N*,*N*,*N*-trioctyloctan-1-ammonium chloride). However, such processes have the disadvantage of generating corrosive halogenated or sulfonate ester intermediates.

An alternative approach to etherification which avoids the formation of these undesirable intermediates is the reaction of an alcohol with an alkene. A well-known example of such an etherification relates to the preparation of methyl or ethyl tert-butyl ether (MTBE or ETBE), which are well known fuel additives. According to Ullmann's Encylopedia of Industrial Chemistry, 2012, Wiley-VCH Verlag GmbH & Co. KGaA, Weinheim, all such MTBE processes: "have in common the reaction of isobutene with a certain molar excess of methanol on a macroporous acidic ion exchanger operating at 50-90 °C". According to Ullmann's, the reasons for using excess alcohol are twofold: (1) the conversion of the alkene to ether is pushed closer to 100% when an excess of the alcohol is used; and (2) excess alcohol suppresses the dimerization and oligomerisation of the alkene, both of which lead to unwanted by-products.

There are examples in the art of MTBE processes where the alkene reactant may be employed in molar excess relative to the alcohol reactant in the etherification, as described, for instance, in GB 1,506,312 and EP 0,249,352. However, where etherification is employed in the preparation of ethers suitable for use as base stocks, as opposed to fuel additives like MTBE or ETBE, there is a distinct prejudice in the art to react the alcohol and alkene with the alcohol component in molar excess, in accordance with the generally accepted method for etherification with alkene and alcohol reactants.

For example, US 2013/0109604 relates to ether compounds for use in improving one or more of solubility and dispersancy of a lubricating oil. According to Example 3 of this document, the alcohol reagent is employed in the etherification reaction in substantial molar excess relative to the alkene reactant. Following the reaction, excess alcohol is removed, along with any unreacted alkene, by means of vacuum distillation at 180 °C. However, yield of the desired ether is relatively low (38%) and the distillation process for isolating the ether product makes the process more energy intensive. Separation by distillation may also be more difficult where the ether formed has a similar boiling point to the excess alcohol reactant used.

US 3 281 475 A discloses (table II) the etherification reaction of n-octyl alcohol with a stoichiometric excess of isobutylene, using Amberlyst 15 as the catalyst. WO 2016/203310 A, WO 2014/207235 A and US 9 193 653 B disclose various ether-based lubricating base oils and base stocks.

Accordingly, there is a need for an alternative etherification process that is suitable for the preparation of ethers for use as lubricating base stocks that avoids the generation of corrosive intermediates and other disadvantages of known etherification processes.

### Summary

Accordingly, in a first aspect, an etherification process is provided for preparing an ether for use as a lubricating base stock, said process comprising contacting an alcohol with an alkene in the presence of an acidic ion-exchange resin etherification catalyst and/or an aluminosilicate etherification catalyst, wherein:
i) the alcohol comprises a C₈₊ aliphatic hydrocarbyl group having the formula (I):

   R-OH (I)

   wherein R is a C₈ to C₄₀ branched-chain alkyl;
ii) the alkene comprises a tertiary olefinic carbon atom; and
ii) the alkene is present in molar excess relative to the alcohol.

In a particularly preferred embodiment, the alcohol has the formula (Ia): 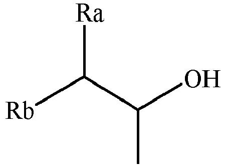 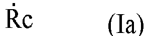 wherein, Rₐ and R_{b} are independently selected from C₃ to C₁₈ alkyl, preferably C₆ to C₁₈ straight-chained alkyl, and R_{c} is selected from Hand C₁ to C₄ alkyl.

In another particularly preferred embodiment, the alkene has the formula (II): 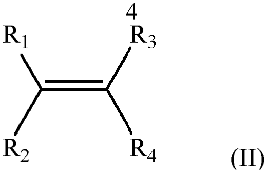 wherein, R₁ and R₂ are independently selected from H, C₁ to C₆ alkyl and C₃ to C₆ cycloalkyl and R₃ and R₄ are independently selected from C₁ to C₆ alkyl and C₃ to C₆ cycloalkyl.

Also provided are processes which, following preparation of the ether compound, include formulation of a lubricating composition comprising the ether compound and use of the resulting lubricant composition for lubricating a surface, such as a surface of an internal combustion engine associated with an automotive vehicle.

### Detailed description

A process is provided for preparing an ether for use as a lubricating base stock, said process comprising contacting an alcohol with an alkene in the presence of an acidic ion-exchange resin etherification catalyst and/or an aluminosilicate etherification catalyst, wherein:
i) the alcohol comprises a C₈₊ aliphatic hydrocarbyl group having the formula (I):

   R-OH (I)

   wherein R is a C₈ to C₄₀ branched-chain alkyl;
ii) the alkene comprises a tertiary olefinic carbon atom; and
iii) the alkene is present in molar excess relative to the alcohol.

For the purposes of the present invention, the following terms as used herein shall, unless otherwise indicated, be understood to have the following meanings:
The term "aliphatic hydrocarbyl" as used herein refers to a group comprising hydrogen and carbon atoms, where one or more carbon atoms may optionally be replaced with -O-, which group may be saturated or unsaturated, preferably saturated, and contains up to 50 carbon atoms. Examples of hydrocarbyl groups include hydrocarbyl groups containing from 8 to 28 carbon atoms, such as from 10 to 26 carbon atoms or from 12 to 24 carbon atoms. Where one or more of the carbon atoms is replaced with -O-, from 2 % to 35 % of the carbon atoms are preferably replaced with -O-, or from 5 % to 25 %. In other examples, the aliphatic hydrocarbyl group has 1 to 3 carbon atoms replaced with -O-, for example 2 carbon atoms replaced with -O-. In other examples, none of the carbon atoms are replaced with -O-.

Examples of aliphatic hydrocarbyl groups include acyclic groups, non-aromatic cyclic groups and groups comprising both an acyclic portion and a non-aromatic cyclic portion. The aliphatic hydrocarbyl group may be straight-chain or branched-chain. The aliphatic hydrocarbyl group includes monovalent groups and polyvalent groups as specified. Examples of monovalent hydrocarbyl groups include alkyl, alkenyl, alkynyl and carbocyclyl (e.g. cycloalkyl or cycloalkenyl).

The term "C₈₊" as used herein refers to a group with 8 or more carbon atoms.

The term "alkyl" as used herein refers to a monovalent straight- or branched-chain alkyl moiety containing from 1 to 40 carbon atoms. Examples of alkyl groups include alkyl groups containing from 1 to 30 carbon atoms, e.g. from 1 to 20 carbon atoms, e.g. from 1 to 14 carbon atoms. Particular examples include alkyl groups containing 1, 2, 3, 4, 5 or 6 carbon atoms. Examples of alkyl groups include methyl, ethyl, n-propyl, isopropyl, n-butyl, sec-butyl, tert- butyl, pentyl, hexyl and the like. Unless specifically indicated otherwise, the term "alkyl" does not include optional substituents.

The term "cycloalkyl" as used herein refers to a monovalent saturated aliphatic hydrocarbyl moiety containing from 3 to 40 carbon atoms and containing at least one ring, wherein said ring has at least 3 ring carbon atoms. The cycloalkyl groups mentioned herein may optionally have alkyl groups attached thereto. Examples of cycloalkyl groups include cycloalkyl groups containing from 3 to 16 carbon atoms, e.g. from 3 to 10 carbon atoms. Particular examples include cycloalkyl groups containing 3, 4, 5 or 6 ring carbon atoms. Examples of cycloalkyl groups include groups that are monocyclic, polycyclic (e.g. bicyclic) or bridged ring system. Examples of cycloalkyl groups include cyclopropyl, cyclobutyl, cyclopentyl, cyclohexyl and the like.

The term "alkenyl" as used herein refers to a monovalent straight- or branched-chain alkyl group containing from 2 to 40 carbon atoms and containing, in addition, at least one carbon-carbon double bond, of either E or Z configuration unless specified. Examples of alkenyl groups include alkenyl groups containing from 2 to 28 carbon atoms, e.g. from 3 to 26 carbon atoms, e.g. from 4 to 24 carbon atoms. Particular examples include alkenyl groups containing 2, 3, 4, 5 or 6 carbon atoms. Examples of alkenyl groups include ethenyl, 2-propenyl, 1-butenyl, 2-butenyl, 3-butenyl, 1-pentenyl, 2-pentenyl, 3-pentenyl, 1-hexenyl, 2-hexenyl, 3-hexenyl and the like.

The term "alkylene" as used herein refers to a divalent straight- or branched-chain saturated hydrocarbyl group consisting of hydrogen and carbon atoms and containing from 1 to 30 carbon atoms. Examples of alkylene groups include alkylene groups that contain from 1 to 20 carbon atoms, e.g. from 1 to 12 carbon atoms, e.g. from 1 to 10 carbon atoms. Particular examples include alkylene groups that contain 1, 2, 3, 4, 5 or 6 carbon atoms.

The term "cycloalkyl-substituted-alkyl" as used herein refers to a straight- or branched-chain alkyl group in which one of the hydrogens of the alkyl chain is replaced with a cycloalkyl group as described hereinabove.

The alcohol employed in the process of the invention has the formula (I):

R-OH (I)

wherein, R is a C₈ to C₄₀ branched-chain alkyl, more preferably a C₁₀ to C₄₀ branched-chain alkyl.

In particularly preferred embodiments, the alcohol employed in the process of the present invention has the formula (Ia): 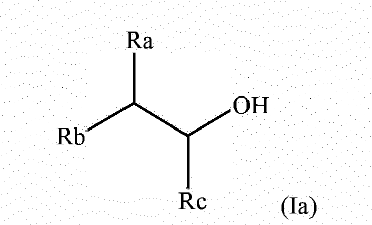 wherein Rₐ and R_{b} are independently selected from C₃ to C₁₈ alkyl and R_{c} is selected from H and C₁ to C₄ alkyl. In preferred embodiments, Rₐ and R_{b} are independently selected from C₆ to C₁₈ straight-chained alkyl.

Preferred examples of the alcohol for use in the process of the present invention include 2-decyl-1-tetradecanol, 2-octyl-1-dodecanol, 2-hexyl-1-decanol and combinations thereof.

In some embodiments, the alkene employed in the process of the invention has the formula (II): 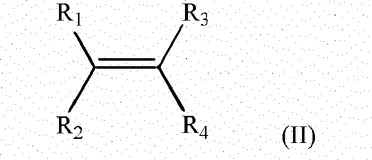 wherein, R₁ and R₂ are independently selected from H, C₁ to C₆ alkyl and C₃ to C₆ cycloalkyl and R₃ and R₄ are independently selected from C₁ to C₆ alkyl and C₃ to C₆ cycloalkyl.

In some embodiments, R₁ and R₂ are independently selected from H and C₁ to C₄ alkyl and R₃ and R₄ are independently selected from C₁ to C₄ alkyl. Preferably, at least one of R₁ and R₂ is C₁ to C₄ alkyl.

In preferred embodiments, R₁ and R₂ are independently selected from Hand C₁ to C₂ alkyl and R₃ and R₄ are independently selected from C₁ to C₂ alkyl. More, preferably at least one of R₁ and R₂ is C₁ to C₂ alkyl,

Preferred examples of the alkene include iso-butene, 2,3-dimethyl-2-butene, 2,3-dimethyl-1-butene, 2-methyl-2-butene, 2-methyl-1-butene and combinations thereof.

In some embodiments, the ether formed by the process of the invention has a total number of carbon atoms of from 20 to 50, preferably from 22 to 40, more preferably from 24 to 30 carbon atoms, and most preferably from 28 to 30 carbon atoms.

The ether compounds described herein may be used to improve the dispersancy properties (for example, by improving soot and sludge dispersancy) and/or viscosity profile (for example, by decreasing deposit forming tendency and/or reducing oxidatively induced thinkening) of a lubricant composition, such as a lubricant composition for an internal combustion engine, preferably associated with an automotive vehicle.

Ether compounds which may be prepared in accordance with the process of the present invention may be particularly suited for blending into a lubricant composition. In particular, such ether compounds may be miscible with conventional base stocks, including hydrocarbon base stocks, as well as with conventional lubricant additives. Moreover, such ether compounds may be used in a lubricant composition in a relatively high amount (for example, in an amount of greater than about 10 % by weight, such as greater than about 20 % by weight or greater than about 30 % by weight) whilst meeting elastomer compatibility requirements for lubricant compositions.

The process of the present invention may be utilized in order to prepare ether compounds from a wide range of commercially available alcohol and alkene feedstocks.

In some embodiments, the compounds are prepared from bio-derived feedstocks. For instance, the resulting ether compounds may contain greater than about 50 %, such as greater than about 70 %, or greater than about 80 % by weight of biobased carbon. The biobased carbon content of the compounds may be measured according to ASTM D6866.

In accordance with the process of the present invention, the alcohol and alkene reactants are contacted in the presence of an acidic ion-exchange resin etherification catalyst and/or an aluminosilicate etherification catalyst. The contacting step may be conducted by any suitable means of which the skilled person is familiar. For instance, the reactants may be contacted within a reactor and may be fed into the reactor either separately or pre-mixed. The reactants may initially all contact the solid catalyst at the same portion of the solid catalyst, or they may be added at different positions of the solid catalyst. The initial point of contact of the reactants with the solid catalyst is the point at which the reactants initially contact each other in the presence of the solid catalyst. The reactants may flow co-currently or countercurrently over the solid catalyst.

The process of the present invention may be carried out in any suitable heterogeneous catalytic reactor, in particular the known types of liquid-phase reactors (including but not limited to plug flow, continuously stirred tank, loop reactors or combinations thereof). Reactive separations, such as catalytic distillation, can also be employed in accordance with the present invention, which may be useful in a continuous process where production and removal of products occurs simultaneously.

The contacting step is performed at a temperature which is suitable for achieving at least partial conversion of the alcohol reactant to ether and which does not exceed the temperature limit at which the catalyst remains stable. In particular, high temperatures can lead to ion-exchange resin decomposition and leaching which is undesirable. A suitable range of temperatures for use in connection with the process of the present invention is from 30 °C to 120 °C. In preferred embodiments, the reactants are contacted at a temperature of from 50 °C to 100 °C, more preferably from 70 °C to 90°C, for example 80 °C.

The contacting step may be performed over a range of pressures. A suitable range of pressures for use in connection with the present invention is from 50 kPa to 5,000 kPa. In preferred embodiments, the reactants are contacted at a pressure from 100 kPa to 1,000 kPa, more preferably from, 100 kPa to 500 kPa, for example from 100 kPa to 250 kPa.

In preferred embodiments, the alkene and alcohol reactants are contacted in the liquid phase. Optionally, solvents may be used for diluting the reactant stream, provided they do not negatively impact the etherification reaction. Suitable solvents include aprotic, hydrocarbon solvents such as pentane, heptane and/or toluene.

The flow rate, in terms of Liquid Hourly Space Velocity (LHSV) (volume of liquid feed stream /total volume of etherification catalyst/hour), at which a pre-mixed alcohol/alkene reactant stream is contacted with the ion-exchange resin etherification catalyst is suitably in the range of from 0.1 to 50 h⁻¹. Higher LHSV is, nevertheless, preferred for reducing the likelihood of alkene dimerization/oligomerisation. Therefore, in preferred embodiments, the LHSV is in the range of from 5 to 50 h⁻¹, more preferably 8 to 30 h⁻¹, even more preferably 10 to 25 h⁻¹.

Following completion of the reaction, the product ether may be isolated from the reaction mixture by known separation processes including filtration, chromatography (e.g. flash column chromatography) and/or distillation. In some embodiments, a distillation column is used to isolate the product ether following the reaction. The distillation may be performed in a conventional distillation column with a number of stages (e.g. ideal stages) commensurate with the reflux ratio required, for example between about 5 and about 50 ideal separation stages.

The distillation column may be operated at atmospheric pressure or sub-atmospheric pressure (vacuum distillation). For example, where distillation is, for instance, used for separation of residual alkene reactant, atmospheric pressures may be used and temperatures of, for example, up to 90 °C or up to 80 °C. In another example, where distillation is, for instance, used for separation of residual alcohol, sub-atmospheric pressures may be used. For example, sub-atmospheric pressures of from 0.1 kPa to 50 kPa, preferably from 0.1 to 10 kPa, more preferably from 0.2 to 2.0 kPa, may be used. Where sub-atmospheric pressures are used, temperatures of, for example, up to 300 °C, preferably up to 275 °C, may be used. In one example, a temperature of from 250 °C to 275 °C is used for the sub-atmospheric pressure distillation.

A particular advantage of the present invention is that by having the alkene reactant in molar excess relative to the alcohol reactant, conversion of the alcohol reactant to product is higher and therefore the amount of residual, unreacted alcohol in the reaction mixture can be reduced, in comparison to where equimolar amounts of, or excess of, alcohol reactant is used relative to the alkene reactant. Reducing the amount of residual alcohol remaining after the etherification reaction is beneficial for isolation of the ether product, particularly where a relatively large alcohol reactant in terms of carbon number is employed in accordance with the present invention, which has a correspondingly large boiling point. Having an alkene present in large excess is also beneficial for driving the reaction equilibrium towards production of the ether product.

Thus in preferred embodiments, the alkene to alcohol molar ratio is at least 2:1, more preferably at least 4:1, even more preferably at least 6:1, and most preferably at least 8 : 1.

In other preferred embodiments, the alkene to alcohol molar ratio is not more than 40 : 1, more preferably not more than 20 : 1, even more preferably not more than 16 : 1, and most preferably not more than 12 : 1.

In particularly preferred embodiments, the alkene to alcohol molar ratio is from 2 : 1 to 40 : 1, more preferably from 4 : 1 to 20 : 1, even more preferably from 6 : 1 to 16 : 1, and most preferably from 8 : 1 to 12 : 1.

The size of the alcohol used in accordance with the present invention is necessary for preparing an ether which is useful as a lubricating base stock. However, it has been found by the inventors that the boiling point of the product ether can be similar to that of the alcohol reactant which can make isolation of the ether product from residual alcohol significantly more onerous than in other etherification processes. For example, the difference in the boiling points of the alcohol and the ether obtained from the process of the invention may be less than 50 °C, less than 20 °C, or even less than 10 °C. Use of a relatively small alkene reactant (in terms of carbon number relative to the alcohol reactant - e.g. a C₄ to C₆ alkene) in the etherification affords a significantly unsymmetrical ether product that is particularly suitable for use as a lubricating base stock. However, use of such a relatively small alkene reactant can exacerbate the problem of separation of the ether product from residual alcohol reactant. Where such a relatively small alkene reactant is employed, the difference in the boiling points of the alcohol reactant and the product ether is likely to be even smaller.

Where a significant amount of residual alcohol remains, for instance, as a result of the use of a molar excess of alcohol reactant in the etherification, this can significantly inhibit the yield of the isolated ether product obtained by distillation, particularly when azeotroping effects with the residual alcohol reactant may also be possible. Therefore, reducing residual alcohol in the etherification product mixture, particularly where a relatively small alkene reactant (e.g. having from 4 to 6 carbon atoms) is used, can make the distillation process less onerous and less energy and/or capital intensive, for example by obviating the requirements for larger distillation columns and/or more ideal separation stages for the distillation.

In preferred embodiments, the process of the present invention does not employ distillation in order to provide an ether in a form for use as a lubricating base stock. In particular, conversion of the alcohol reactant may be high enough that it is not necessary to remove any residual alcohol before using the ether when blending a lubricating composition. For example, the residual alcohol content may be less than 20 wt.%, less than 15 wt.%, less than 10 wt.%, or even less than 5 wt.%, based on the combination of ether product and residual alcohol. Thus, in some embodiments, the process of the present invention may further include the step of blending the ether product obtained from the process into a lubricant composition, without an intermediate residual alcohol removal step. As will be appreciated, this does not preclude intermediate steps prior to blending to remove solvent(s) and/or lighter hydrocarbons present in the reaction mixture.

The process of the present invention therefore represents a means of preparing an ether which is useful as a lubricating base stock which avoids the formation of corrosive intermediates and which may be isolated from the reaction mixture more readily. By operating the etherification process in accordance with the present invention, it is possible to provide good conversion of the alcohol to the ether product, thereby reducing and/or eliminating the unreacted alcohol in the reaction mixture.

The acidic ion-exchange resin etherification catalyst used in accordance with the invention is also known as a cationic exchange resin. Suitable forms of ion exchange resin for use in the process of the invention include acidic macroreticular-type cation exchange resin or an acidic gel-type cation exchange resin.

Typically, the acidic gel-type cation exchange resins that may be used are based on an insoluble cross-linked polymeric matrix, typically having a pore diameter of at most 30 Å. In preferred embodiments, the acidic gel-type cation exchange resins are based on a cross-linked polystyrene based matrix, preferably having a pore diameter of at most 30 Å. More preferably, the acidic gel-type cation exchange resins that may be used are based on a cross-linked polymeric matrix prepared by copolymerising styrene and divinyl benzene and preferably having a pore diameter of at most 30 Å.

In preferred embodiments, the acidic gel-type cation exchange resins are strong acid ion exchange resins, such as sulfonated resins. In particular, the acidic gel-type cation exchange resins are preferably based on a sulfonated insoluble cross-linked polymeric matrix preferably having a pore diameter of at most 30 Å.

In a particularly preferred embodiment, the acidic gel-type cation exchange resin used is based on a sulfonated copolymer of styrene and divinyl benzene, preferably having a pore diameter of at most 30 Å.

Examples of suitable acidic gel-type cation exchange resins include, but are not limited to, the strong acid Dowex (trademark) gel-type ion exchange resins, the strong acid Amberlyst (trademark) gel-type ion exchange resins, the strong acid Diaion (trademark) gel-type ion exchange resins, the strong acid Lewatit (trademark) gel-type ion exchange resins, the strong acid Purolite (trademark) gel-type ion exchange resins, the strong acid gel-type ion exchange resins available from ResinTech Inc., and mixtures thereof.

The acidic macroreticular-type cation exchange resins useful in the present invention are typically based on an insoluble cross-linked polymeric matrix typically having a pore diameter in the range of from 50 to 1,000,000 Å. In preferred embodiments, the acidic macroreticular-type cation exchange resins used in the process of the present invention are based on a cross-linked polystyrene based matrix having a pore diameter in the range of from 50 to 1,000,000 Å. More preferably, the acidic macroreticular-type cation exchange resins useful in the process of the present invention are based on a cross-linked polymeric matrix prepared by copolymerising styrene and divinyl benzene, having a pore diameter in the range of from 50 to 1,000,000 Å.

In preferred embodiments, the acidic macroreticular-type cation exchange resins used in the process of the present invention are strong acid ion exchange resins, such as sulfonated resins. In particular, the acidic macroreticular-type cation exchange resins used in the process of the present invention are preferably based on a sulfonated insoluble cross-linked polymeric matrix preferably having a pore diameter in the range of from 50 to 1,000,000 Å. Thus, in a particularly preferred embodiment, the acidic macroreticular-type cation exchange resins used in the process of the present invention are sulfonated copolymers of styrene and divinyl benzene, having a pore diameter in the range of from 50 to 1,000,000 Å. Suitable acidic macroreticular-type cation exchange resins include, but are not limited to, the strong acid Dowex macroreticular-type ion exchange resins, the strong acid Amberlyst macroreticular-type ion exchange resins, the strong acid Diaion macroreticular-type ion exchange resins, the strong acid Lewatit macroreticular-type ion exchange resins, the strong acid Purolite macroreticular-type ion exchange resins, the strong acid macroreticular-type ion exchange resins available from ResinTech Inc., and mixtures thereof.

A single acidic ion-exchange resin may be used in the process of the present invention or combinations of different ion-exchange resins may be employed, as desired.

An acidic ion-exchange resin may be used alone or in combination with an aluminosilicate etherification catalyst.

The aluminosilicate etherification catalyst which may be used in accordance with the present invention is a solid porous material having acid sites or acid functionality. The aluminosilicate may thus optionally be treated or impregnated with an acid, such as phosphoric acid, phosphonic acid, sulfuric acid or a sulphonic acid. The aluminosilicate etherification catalyst used in accordance with the invention may be a microporous aluminosilicate or a mesoporous aluminosilicate.

In preferred embodiments, the aluminosilicate etherification catalyst is a zeolite. The zeolite may have at least one channel defined by a 10-membered or 12-membered ring.

The zeolite may be a large-pore zeolite having at least one channel having a diameter of at least 5 Å, at least 6 Å, or at least 7 Å.

In other embodiments, the zeolite has the framework type BEA, MFI, FAU or MOR, and therefore includes beta, pentasil, faujasite and mordenite zeolites. Examples of faujasites are zeolite Y and zeolite X.

The zeolite is preferably in the acidic (H) form. Thus, the zeolites include, for example, H-faujasites and H-mordenites, and the zeolite may be zeolite H-Y or zeolite H-X.

The density of acid sites in zeolites is dependent on the silica to alumina ratio (SAR) of the zeolite. The lower the SAR value the greater the proportion of aluminium atoms and the greater the density of acidic sites. Thus, for optimum etherification performance it is preferred to use zeolites having low SAR values. Thus, in some embodiments, the zeolite has an SAR of at most 100, for example in the range of from 1 to 100, in other embodiments has an SAR of at most 50, for example in the range of from 1 to 50, in further embodiments has an SAR of at most 20, for example in the range of from 1 to 20, in still further embodiments has an SAR of at most 15, for example in the range of from 1 to 15, and in yet further embodiments has an SAR of at most 10, for example in the range of from 1 to 10.

In accordance with another embodiment, the process of the invention also further comprises blending the ether obtained from the process into a lubricant composition, for instance by blending the ether with one or more additional base stocks and/or one or more lubricant additives. The ether obtained from the process of the invention may be miscible with conventional base stocks, including hydrocarbon base stocks, as well as with conventional lubricant additives. Moreover, such ether compounds may be used in a lubricant composition in a relatively high amount (for example, in an amount of greater than about 10 % by weight, such as greater than about 20 % by weight or greater than about 30 % by weight).

Base stocks other than the ether compound formed in the process of the present invention which are suitable for use blending for preparing a lubricant composition include non-aqueous base stocks, such as Group I, Group II, Group III, Group IV and Group V base stocks.

The lubricant composition may comprise a single lubricant additive, though it will typically comprise a combination of lubricant additives. The lubricant additives will typically be present in the lubricant composition in an amount of from about 5 % to about 40 % by weight, such as about 10 % to about 30 % by weight.

Suitable lubricant additives include detergents (including metallic and non- metallic detergents), friction modifiers, dispersants (including metallic and non-metallic dispersants), viscosity modifiers, dispersant viscosity modifiers, viscosity index improvers, pour point depressants, anti-wear additives, rust inhibitors, corrosion inhibitors, antioxidants (sometimes also called oxidation inhibitors), anti-foams (sometimes also called anti-foaming agents), seal swell agents (sometimes also called seal compatibility agents), extreme pressure additives (including metallic, non-metallic, phosphorus containing, non-phosphorus containing, sulphur containing and non-sulphur containing extreme pressure additives), surfactants, demulsifiers, anti-seizure agents, wax modifiers, lubricity agents, anti-staining agents, chromophoric agents, metal deactivators, and mixtures of two or more thereof.

In some embodiments, the lubricant composition comprises a detergent. Examples of detergents include ashless detergents (that is, non-metal containing detergents) and metal-containing detergents. Suitable non-metallic detergents are described for example in US 7,622,431. Metal-containing detergents comprise at least one metal salt of at least one organic acid, which is called soap or surfactant. Suitable organic acids include for example, sulphonic acids, phenols (suitably sulphurised and including for example, phenols with more than one hydroxyl group, phenols with fused aromatic rings, phenols which have been modified for example, alkylene bridged phenols, and Mannich base-condensed phenols and saligenin-type phenols, produced for example by reaction of phenol and an aldehyde under basic conditions) and sulphurised derivatives thereof, and carboxylic acids including for example, aromatic carboxylic acids (for example hydrocarbyl-substituted salicylic acids and derivatives thereof, for example hydrocarbyl substituted salicylic acids and sulphurised derivatives thereof).

In some embodiments, the lubricant composition comprises a friction modifier. Suitable friction modifiers include for example, ash-producing additives and ashless additives. Examples of suitable friction modifiers include fatty acid derivatives including for example, fatty acid esters, amides, amines, and ethoxylated amines. Examples of suitable ester friction modifiers include esters of glycerol for example, mono-, di-, and tri-oleates, mono-palmitates and mono-myristates. A particularly suitable fatty acid ester friction modifier is glycerol monooleate. Examples of suitable friction modifiers also include molybdenum compounds for example, organo molybdenum compounds, molybdenum dialkyldithiocarbamates, molybdenum dialkylthiophosphates, molybdenum disulphide, tri-molybdenum cluster dialkyldithiocarbamates, non-sulphur molybdenum compounds and the like. Suitable molybdenum-containing compounds are described for example, in EP 1533362 Al for example in paragraphs [0101] to [0117].

In some embodiments, the lubricant composition comprises a dispersant. Examples of suitable ashless dispersants include oil soluble salts, esters, amino-esters, amides, imides and oxazolines of long chain hydrocarbon-substituted mono- and polycarboxylic acids or anhydrides thereof; thiocarboxylate derivatives of long chain hydrocarbons; long chain aliphatic hydrocarbons containing polyamine moieties attached directly thereto; Mannich condensation products formed by condensing a long chain substituted phenol with formaldehyde and polyalkylene polyamine; Koch reaction products and the like.

In some embodiments, the lubricant composition comprises a dispersant viscosity modifier. Examples of suitable dispersant viscosity modifiers and methods of making them are described in WO 1999/021902, WO 2003/099890 and WO 2006/099250.

In some embodiments, the lubricant composition comprises a viscosity index improver. Examples of suitable viscosity modifiers include high molecular weight hydrocarbon polymers (for example polyisobutylene, copolymers of ethylene and propylene and higher alpha-olefins); polyesters (for example polymethacrylates); hydrogenated poly(styrene-co-butadiene or isoprene) polymers and modifications (for example star polymers); and esterified poly(styrene-co-maleic anhydride) polymers. Oil- soluble viscosity modifying polymers generally exhibit number average molecular weights of at least about 15000 to about 1000000, such as about 20000 to about 600000 as determined by gel permeation chromatography or light scattering methods.

In some embodiments, the lubricant composition comprises a pour point depressant. Examples of suitable pour point depressants include C₈ to C₁₈ dialkyl fumarate/vinyl acetate copolymers, methacrylates, polyacrylates, polyarylamides, polymethacrylates, polyalkyl methacrylates, vinyl fumarates, styrene esters, condensation products of haloparaffin waxes and aromatic compounds, vinyl carboxylate polymers, terpolymers of dialkyfumarates, vinyl esters of fatty acids and allyl vinyl ethers, wax naphthalene and the like. In at least some examples, the at least one lubricant additive includes at least one anti-wear additive. Examples of suitable anti-wear additives include non-phosphorus containing additives for example, sulphurised olefins. Examples of suitable anti-wear additives also include phosphorus-containing anti-wear additives. Examples of suitable ashless phosphorus-containing anti-wear additives include trilauryl phosphite and triphenylphosphorothionate and those disclosed in paragraph [0036] of US 2005/0198894. Examples of suitable ash-forming, phosphorus-containing anti-wear additives include dihydrocarbyl dithiophosphate metal salts. Examples of suitable metals of the dihydrocarbyl dithiophosphate metal salts include alkali and alkaline earth metals, aluminium, lead, tin, molybdenum, manganese, nickel, copper and zinc. Particularly suitable dihydrocarbyl dithiophosphate metal salts are zinc dihydrocarbyl dithiophosphates (ZDDP).

In some embodiments, the lubricant composition comprises a rust inhibitor. Examples of suitable rust inhibitors include non-ionic polyoxyalkylene polyols and esters thereof, polyoxyalkylene phenols, polyoxyalkylene polyols, anionic alky sulphonic acids, zinc dithiophosphates, metal phenolates, basic metal sulphonates, fatty acids and amines.

In some embodiments, the lubricant composition comprises a corrosion inhibitor. Examples of suitable corrosion inhibitors include phosphosulphurised hydrocarbons and the products obtained by the reaction of phosphosulphurised hydrocarbon with an alkaline earth metal oxide or hydroxide, non-ionic polyoxyalkylene polyols and esters thereof, polyoxyalkylene phenols, thiadiazoles, triazoles and anionic alkyl sulphonic acids. Examples of suitable epoxidised ester corrosion inhibitors are described in US 2006/0090393.

In some embodiments, the lubricant composition comprises an antioxidant. Examples of suitable antioxidants include alkylated diphenylamines, N-alkylated phenylenediamines, phenyl-a-naphthylamine, alkylated phenyl-a-naphthylamines, dimethylquinolines, trimethyldihydroquinolines and oligomeric compositions derived therefrom, hindered phenolics (including ashless (metal-free) phenolic compounds and neutral and basic metal salts of certain phenolic compounds), aromatic amines (including alkylated and non-alkylated aromatic amines), sulphurised alkyl phenols and alkali and alkaline earth metal salts thereof, alkylated hydroquinones, hydroxylated thiodiphenyl ethers, alkylidenebisphenols, thiopropionates, metallic dithiocarbamates, 1,3,4-dimercaptothiadiazole and derivatives, oil soluble copper compounds (for example, copper dihydrocarbyl thio- or thio-phosphate, copper salts of a synthetic or natural carboxylic acids, for example a C₈ to C₁₈ fatty acid, an unsaturated acid or a branched carboxylic acid, for example basic, neutral or acidic Cu(I) and/or Cu(II) salts derived from alkenyl succinic acids or anhydrides), alkaline earth metal salts of alkylphenolthioesters, suitably containing C₅ to C₁₂ alkyl side chains, calcium nonylphenol sulphide, barium t-octylphenyl sulphide, dioctylphenylamine, phosphosulphised or sulphurised hydrocarbons, oil soluble phenates, oil soluble sulphurised phenates, calcium dodecylphenol sulphide, phosphosulphurised hydrocarbons, sulphurised hydrocarbons, phosphorus esters, low sulphur peroxide decomposers and the like.

In some embodiments, the lubricant composition comprises an antifoam agent. Examples of suitable anti-foam agents include silicones, organic polymers, siloxanes (including poly siloxanes and (poly) dimethyl siloxanes, phenyl methyl siloxanes), acrylates and the like.

In some embodiments, the lubricant composition comprises a seal swell agent. Examples of suitable seal swell agents include long chain organic acids, organic phosphates, aromatic esters, aromatic hydrocarbons, esters (for example butylbenzyl phthalate) and polybutenyl succinic anhydride.

The lubricant composition may comprise lubricant additives in the amounts shown in Table 2.

**Table 2**

| | Lubricant composition | |
|---|---|---|
| Additive type | Suitable amount (actives) if present by weight | Preferred amount (actives) if present by weight |
| Phosphorus-containing anti-wear additives | Corresponding to about 10 to about 6000 ppm P | Corresponding to about 10 to about 1000 ppm P |
| Molybdenum-containing anti-wear additives | Corresponding to about 10 to about 1000 ppm Mo | Corresponding to about 40 to about 600 ppm Mo |
| Boron-containing anti-wear additives | Corresponding to about 10 to about 500 ppm B | Corresponding to about 50 to about 100 ppm B |
| Friction modifiers | About 0.01 to about 5 % | About 0.01 to about 1.5 % |
| Molybdenum-containing friction modifiers | Corresponding to about 10 to about 1000 ppm Mo | Corresponding to about 400 to about 600 ppm Mo |
| Dispersants | About 0.1 to about 20 % | About 0.1 to about 8 % |
| Detergents | About 0.01 to about 6 % | About 0.01 to about 4 % |
| Viscosity index improvers | About 0.01 to about 20 % | About 0.01 to about 15 % |
| Pour point depressants | About 0.01 to about 5 % | About 0.01 to about 1.5 % |
| Corrosion and/or rust inhibitors | About 0.01 to about 5 % | About 0.01 to about 1.5 % |
| Anti-oxidants | About 0.01 to about 10 % | About 0.5 to 5 about % |
| Antifoams containing silicon | Corresponding to about 1 to about 20 ppm Si | Corresponding to about 1 to about 10 ppm Si |

The lubricant compositions preparable in accordance with the present invention may have a kinematic viscosity at 40 °C of less than about 60 cSt, such as less than about 55 cSt, or less than about 50 cSt. The lubricant compositions may have a kinematic viscosity at 100 °C of less than about 12 cSt, such as less than about 10 cSt, or less than about 9.5 cSt. The lubricant compositions may have a viscosity index of greater than about 100, such as greater than about 110, or greater than about 120. The kinematic viscosity at 40 °C and the kinematic viscosity at 100 °C may be measured according to ASTM D445. The viscosity index may be calculated according to ASTM D2270.

The lubricant compositions may have a Noack volatility of less than about 25 %, such as less than about 15 %, or less than about 10 % by weight. Noack volatility may be measured according to CEC-L-40-A-93.

The lubricant compositions may have a viscosity at 150 °C and a shear rate of 10⁶ s⁻¹ of no greater than 3 cP, such as no greater than 2.8 cP. This high temperature high shear viscosity may be measured according to CEC-L-36-A-90.

The lubricant compositions may have at least one of:
an oxidative stability performance on a CEC-L-088-02 test indicated by an absolute viscosity increase at 40 °C of no more than 45 cSt, such as no more than 35 cSt or no more than 25 cSt; a fuel economy performance on a CEC-L-054-96 test of at least 2.5 %, such as at least 3 %; and a piston cleanliness performance on a CEC-L-088-02 test indicated by an overall piston merit of at least 8.5, such as 9.

The lubricant compositions may have a cold-crankcase simulator performance at -30 °C of less than about 3000, such as less than about 2800, or less than about 2750, for example as measured according to ASTM D5293.

Preferred lubricant compositions meet the requirements set out in SAE J300.

In a yet further embodiment of the invention, after the lubricant composition has been prepared, the process may further comprise lubricating a surface with the lubricant composition by supplying the lubricant composition to a surface for lubrication.

Suitable surfaces include those in power transmission systems for example drive lines and gear boxes for example for vehicles including for example passenger vehicles and heavy duty vehicles; and those in internal combustion engines, for example the crankcases of internal combustion engines. Suitable surfaces also include those in turbine bearings for example in water turbine bearings.

Suitable internal combustion engines include, for example, engines used in automotive applications, engines used in marine applications and engines used in land- based power generation plants. The lubricant compositions are particularly suited to use in an automotive internal combustion engine.

The invention will now be described with reference to the accompanying figures and examples, which are not limiting in nature, in which:
**Fig. 1** is a gas chromatography spectrum for a crude reaction mixture following the etherification reaction according to Example 1; and
**Fig. 2** is a gas chromatography spectrum for a crude reaction mixture following the etherification reaction according to Example 2.

### Examples

### Example 1 - 3-(((2,3-dimethylbutan-2-yl)oxy)methyl)heptane

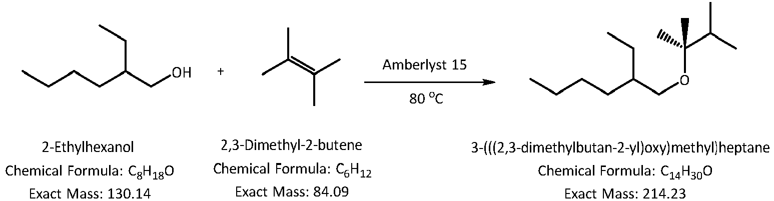

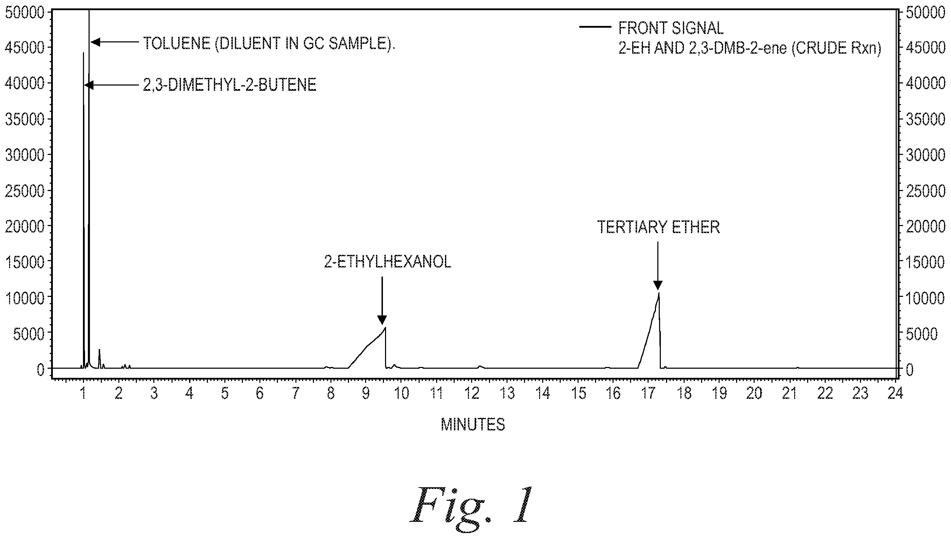

A solution of 2-ethylhexanol (10 g, 0.077 mol) and 2,3-dimethyl-2-butene (13 g, 0.154 mol) was treated with Amberlyst 15 resin (300 mg (dry mass), pre-washed in deionised water and dried) and stirred at 80 °C for 24 h in a flask fitted with a reflux condensor. The reaction mixture was sampled and analysed using gas chromatography (GC), the results of which are shown in the GC trace corresponding to Figure 1. The reaction mixture was then filtered to remove the Amberlyst 15 resin and distilled *in vacuo* to yield recovered 2-ethylhexanol (4.1 g, 0.032 mol, distilled at 48-52 °C, 1.8 mbar (0.18 kPa)) and tertiary ether product (9.2 g, 0.043 mol, distilled at 68-70 °C, 1.8 mbar (0.18 kPa)).

### Example 2 - 11-(((2,3-dimethylbutan-2-yl)oxy)methyl)tricosane

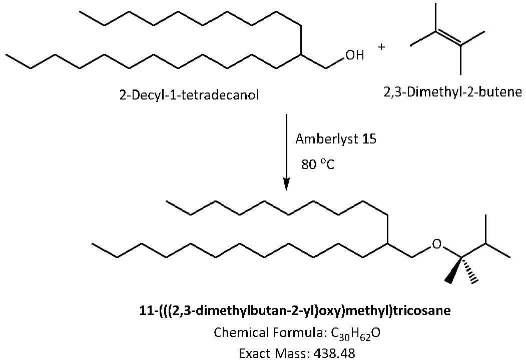

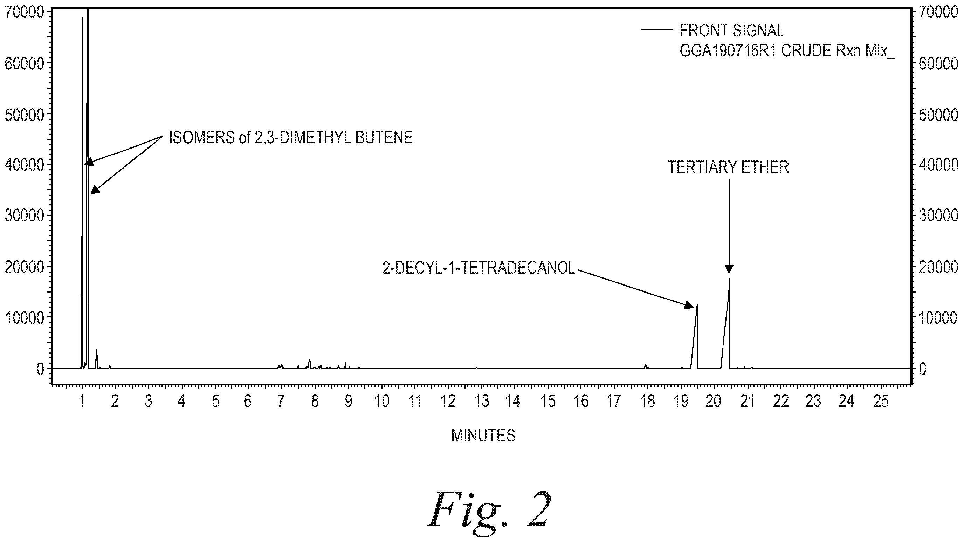

A solution of 2-decyl-1-tetradecanol (5 g, 0.014 mol) and 2,3-dimethyl-2-butene (16 g, 0.190 mol) was treated with Amberlyst 15 resin (200 mg (dry mass), pre-washed in deionised water and dried) and stirred at 80 °C for 24 h in a flask fitted with a reflux condensor. Progress of the reaction was monitored by analysing a sample using gas chromatography (GC) after 2 h of reaction and is shown in Figure 2. After this time the reaction mixture was filtered to remove the resin and purified by flash column chromatography using heptane as the eluent to yield the tertiary ether (4.2 g, 0.01 mol, 68 % yield) as a colourless oil.

### Examples 3 to 8 and Comparative Examples A to C - 11-((tert-pentyloxy)methyl)tricosane

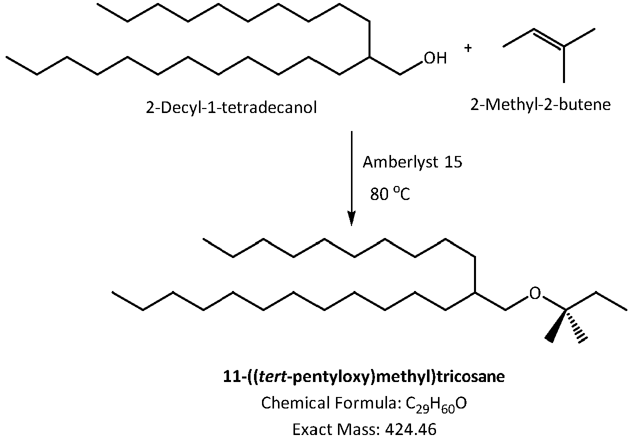

### General Method

Solutions of 2-decyl-1-tetradecanol, 2-methyl-2-butene and vary amounts of toluene solvent were passed over a bed of Amberlyst 15 resin (2.5 mL bed length) at 60 °C, 10 barg (1,000 kPa.g) and at a flow rate (LHSV) of 12, 24 or 48 h⁻¹. The reactions took place in the liquid phase. Different molar ratios of alcohol and alkene were investigated. Products of each of the reactions were analysed by gas chromatography (GC) and results are provided in Table 3 below.

**Table 3**

| **Example** | **Alcohol (mol eq.)** | **Alkene (mol eq.)** | **Toluene (mol eq.)** | **LHSV (h⁻¹)** | **C₅^{*} (Mol.%)** | **C₁₀^{**} (Mol.%)** | **Alcohol (Mol.%)** | **Ether (Mol.%)** | **Ether Yield from alcohol (%)** | **Alkene Conversion (%)** | **Ether selectivity from Alkene (%)** |
|---|---|---|---|---|---|---|---|---|---|---|---|
| A | 1 | 1 | 9 | 24 | 41 | 1 | 51 | 6 | 10 | 15 | 81 |
| 3 | 1 | 1.25 | 8.75 | 24 | 45 | 2 | 45 | 8 | 15 | 18 | 82 |
| 4 | 1 | 2 | 8 | 24 | 48 | 3 | 40 | 9 | 18 | 19 | 73 |
| 5 | 1 | 5 | 5 | 24 | 67 | 10 | 9 | 14 | 60 | 26 | 58 |
| 6 | 1 | 10 | 0 | 24 | 57 | 27 | 3 | 12 | 78 | 41 | 31 |
| 7 | 1 | 10 | 0 | 12 | 55 | 29 | 3 | 13 | 79 | 43 | 31 |
| 8 | 1 | 10 | 0 | 48 | 78 | 6 | 8 | 9 | 52 | 16 | 61 |
| B | 0 | 1 | 10 | 24 | 100 | 0.1 | n/a | n/a | n/a | 0.1 | 0 |
| C | 0 | 1 | 10 | 48 | 100 | 0.1 | n/a | n/a | n/a | 0.1 | 0 |

| | | | | | | | | | | | |
|---|---|---|---|---|---|---|---|---|---|---|---|
| * - corresponds to unreacted alkene reactant ** - corresponds to dimerization by-products | | | | | | | | | | | |

The results in Table 3 illustrate the benefits of the invention. The yield of the product ether from the alcohol increases as the number of equivalents of alkene increases. Yield of ether from the alcohol is increased especially where the ratio of alkene to alcohol is greater than 2 : 1, as in Examples 5 to 8.

### Example 9 - Assessing properties of ether base stocks

The following properties of the ether base stocks prepared in Examples 2 and 3-8, namely 11-(((2,3-dimethylbutan-2-yl)oxy)methyl)tricosane and 11-((tert-pentyloxy)methyl)tricosane, were tested:
Kinematic viscosity at 100 °C (KV100) and kinematic viscosity at 40 °C (KV40) were tested according to ASTM D7279.
Viscosity index (VI) was calculated according to ASTM D2270.
Pour point was determined according to ASTM D7346.

Differential scanning calorimetry (DSC) oxidation onset temperature was tested using a method which was based on ASTM E2009 (method B). According to the method, the base stocks were heated from 50 °C to 300 °C, at a rate of 50 °C / minute, under a pressure of 500 psi in an aluminium SFI pan. The temperature at which an exotherm was observed was recorded.

Noack volatility was measured for 11-((tert-pentyloxy)methyl)tricosane in accordance with CEC-L-40-A-93.

The results of the tests are summarized in Table 4, together with results obtained from conventional base stocks (Durasyn 162, a group IV base stock; Durasyn 164, a group IV base stock; Yubase 3, a group II base stock; Yubase 4, a group III base stock; Yubase 4 Plus, a group III base stock; Nexbase 3020, a group II base stock; Nexbase 3030, a group II base stock; Nexbase 3043, a group III base stock; and Chevron 100RLV, a group II base stock).

**Table 4**

| | **KV100 (cSt)** | **KV40 (cSt)** | **VI** | **Pour Point (°C)** | **DSC Oxidation Onset T (°C)** | **Noack (% by weight)** |
|---|---|---|---|---|---|---|
| 11-((tert-pentyloxy)methyl)tricosane | 3.47 | 14.0 | 128 | -30 | 221.44 | 17.0 |
| 11-(((2,3-dimethylbutan-2-yl)oxy)methyl)tricosane | 3.81 | 16.01 | 132 | -54 | 229.26 | - |
| Durasyn 162 | 1.7 | 5.2 | 92 | -72 | 223.61 | THM |
| Durasyn 164 | 4.0 | 17.8 | 126 | -75 | 221.31 | 13.1 |
| Yubase 3 | 3.0 | 14.1 | 105 | -36 | 220.74 | 40.5 |
| Yubase 4 | 4.2 | 19.2 | 126 | -12 | 220.00 | 14.1 |
| Yubase 4 Plus | 4.2 | 18.4 | 138 | -18 | 220.32 | 12.9 |
| Nexbase 3020 | 2.2 | 7.6 | 93 | -51 | 221.66 | THM |
| Nexbase 3030 | 3.0 | 12.0 | 101 | -39 | 221.05 | 38.1 |
| Nexbase 3043 | 4.3 | 19.9 | 124 | -18 | 222.09 | 14.0 |
| Chevron 110RLV | 4.6 | 22.6 | 119 | -15 | 225.86 | 14.6 |

| | | | | | | |
|---|---|---|---|---|---|---|
| THM = Too high to measure | | | | | | |

The results of the tests shown in Table 4 demonstrate that the properties of the ether base stocks obtainable by the process of the present invention compare favourably with those of conventional base stocks. The ether base stocks obtainable by the process of the present invention may thus be formulated into a lubricant composition for use in lubrication applications.

The dimensions and values disclosed herein are not to be understood as being strictly limited to the exact numerical values recited. Instead, unless otherwise specified, each such dimension is intended to mean both the recited value and a functionally equivalent range surrounding that value. The citation of any document is not an admission that it is prior art with respect to any invention disclosed or claimed herein or that it alone, or in any combination with any other reference or references, teaches, suggests or discloses any such invention. Further, to the extent that any meaning or definition of a term in this document conflicts with any meaning or definition of the same term in a document, the meaning or definition assigned to that term in this document shall govern.

While particular embodiments of the present invention have been illustrated and described, it would be obvious to those skilled in the art that various other changes and modifications can be made without departing from the scope of the invention.

## Claims

1. A process for preparing an ether, said process comprising contacting an alcohol with an alkene in the presence of an acidic ion-exchange resin etherification catalyst and/or an aluminosilicate etherification catalyst, wherein:
i) the alcohol comprises a C₈₊ aliphatic hydrocarbyl group having the formula (I):
R-OH (I)
wherein R is a C₈ to C₄₀ branched-chain alkyl;
ii) the alkene comprises a tertiary olefinic carbon atom; and
iii) the alkene is present in molar excess relative to the alcohol.

2. The process of Claim 1, wherein the alkene to alcohol molar ratio is at least 2:1, preferably at least 4 : 1, more preferably at least 6 : 1, most preferably at least 8 : 1.

3. The process of Claim 1 or Claim 2, wherein the alkene to alcohol molar ratio is not more than 40 : 1, preferably not more than 20 : 1, more preferably not more than 16 : 1, and most preferably not more than 12 : 1.

4. The process of any of Claims 1 to 3, wherein the alkene to alcohol molar ratio is from 2 : 1 to 40 : 1, preferably from 4 : 1 to 20 : 1, more preferably from 6 : 1 to 16 : 1, most preferably from 8 : 1 to 12 : 1.

5. The process of Claim 1, wherein R is a C₁₀ to C₄₀ branched-chain alkyl.

6. The process of any one of the preceding claims, wherein the alcohol has the formula (Ia): 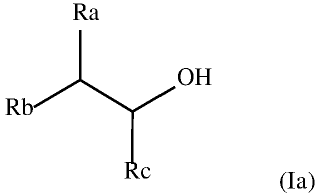 wherein, Rₐ and R_{b} are independently selected from C₃ to C₁₈ alkyl, preferably C₆ to C₁₈ straight-chained alkyl, and R_{c} is selected from H and C₁ to C₄ alkyl.

7. The process of any one of the preceding claims, wherein the alcohol is selected from 2-decyl-1-tetradecanol, 2-octyl-1-dodecanol, 2-hexyl-1-decanol and combinations thereof.

8. The process of any one of the preceding claims, wherein the alkene has the formula (II): 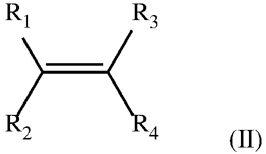 wherein, R₁ and R₂ are independently selected from H, C₁ to C₆ alkyl and C₃ to C₆ cycloalkyl and R₃ and R₄ are independently selected from C₁ to C₆ alkyl and C₃ to C₆ cycloalkyl.

9. The process of Claim 8, wherein R₁ and R₂ are independently selected from H and C₁ to C₄ alkyl, preferably wherein at least one of R₁ and R₂ is C₁ to C₄ alkyl, and R₃ and R₄ are independently selected from C₁ to C₄ alkyl.

10. The process of Claim 9, wherein R₁ and R₂ are independently selected from H and C₁ to C₂ alkyl, preferably wherein at least one of R₁ and R₂ is C₁ to C₂ alkyl, and R₃ and R₄ are independently selected from C₁ to C₂ alkyl.

11. The process of any of the preceding claims wherein the alkene is a C₄ to C₆ alkene.

12. The process of any one of the preceding claims, wherein the alkene is selected from *iso*-butene, 2,3-dimethyl-2-butene, 2,3-dimethyl-1-butene, 2-methyl-2-butene, 2-methyl-1-butene and combinations thereof.

13. The process of any one of the preceding claims, wherein the ether formed in the process has a total number of carbon atoms of from 20 to 50, preferably from 22 to 40, more preferably from 24 to 30 carbon atoms, and most preferably 28 to 30 carbon atoms.

14. The process of any one of the preceding claims, wherein the difference in the boiling points of the alcohol and the ether obtained from the process is less than 50 °C, for example less than 20 °C or less than 10 °C.

15. The process of any one of the preceding claims, wherein the acidic ion-exchange resin is an acidic macroreticular-type ion-exchange resin or an acidic gel-type ion-exchange resin, preferably where the macroreticular-type or gel-type resin is a strong acid resin, such as a sulfonated resin.

16. The process of any one of the preceding claims, wherein the aluminosilicate etherification catalyst is a zeolite, preferably where the zeolite has at least one channel defined by a 10-membered or 12-membered ring.

17. The process of any one of the preceding claims, wherein the aluminosilicate etherification catalyst is zeolite H-Y.

18. The process according to any one of the preceding claims, wherein the reaction is operated with the reactants in the liquid phase.

19. The process of any one of the preceding claims, wherein the reactants are contacted at a temperature in the range of from 30 °C to 120 °C, preferably from 50 °C to 100 °C, more preferably from 70 °C to 90°C, for example 80 °C.

20. The process of any one of the preceding claims, wherein the reactants are contacted at a pressure of from 100 kPa to 1,000 kPa, preferably from 100 kPa to 500 kPa, more preferably from 100 kPa to 250 kPa.

21. The process of any one of the preceding claims, wherein the ether product is isolated from the reaction mixture by means of distillation, preferably where the distillation is operated at sub-atmospheric pressure.

22. A process for preparing a lubricant composition, comprising preparing an ether according to the process of any one of the preceding claims, and blending the ether obtained from the process into a lubricant composition.

23. The process of Claim 22, and to the extent that it depends from any one of Claims 1 to 20, wherein the ether obtained undergoes no intermediate residual alcohol removal step(s) prior to blending the ether into the lubricant composition.

24. The process of Claim 22 or Claim 23, wherein the ether is blended with one or more additional base stocks and/or one or more lubricant additives.

25. The process of any one of Claims 22 to 24, wherein the ether is present in the lubricant composition in an amount of greater than 10 % by weight, preferably greater than 20 % by weight, more preferably greater than 30 % by weight.

26. A process for lubricating a surface, comprising preparing a lubricant composition according to the process of any one of Claims 22 to 25, and supplying the lubricant composition to a surface for lubrication, such as a surface in an internal combustion engine.

## Patentansprüche

1. Verfahren zur Herstellung eines Ethers, wobei das Verfahren ein Inkontaktbringen eines Alkohols mit einem Alken in Gegenwart eines sauren Ionenaustauschharz-Veretherungskatalysators und/oder eines Aluminiumsilicat-Veretherungskatalysators umfasst, wobei:
i) der Alkohol eine aliphatische C₈₊-Hydrocarbylgruppe mit der Formel (I) umfasst:
R-OH (I),
wobei R eine verzweigtkettiges C₈- bis C₄₀-Alkyl ist;
ii) das Alken ein tertiäres olefinisches Kohlenstoffatom umfasst und
iii) das Alken in Bezug auf den Alkohol in einem molaren Überschuss vorliegt.

2. Verfahren nach Anspruch 1, wobei das Molverhältnis von Alken zu Alkohol mindestens 2:1, vorzugsweise mindestens 4:1, mehr bevorzugt mindestens 6:1, am meisten bevorzugt mindestens 8:1 beträgt.

3. Verfahren nach Anspruch 1 oder Anspruch 2, wobei das Molverhältnis von Alken zu Alkohol höchstens 40:1, vorzugsweise höchstens 20:1, mehr bevorzugt höchstens 16:1 und am meisten bevorzugt höchstens 12:1 beträgt.

4. Verfahren nach einem der Ansprüche 1 bis 3, wobei das Molverhältnis von Alken zu Alkohol von 2:1 bis 40:1, vorzugsweise von 4:1 bis 20:1, mehr bevorzugt von 6:1 bis 16:1, am meisten bevorzugt von 8:1 bis 12:1 beträgt.

5. Verfahren nach Anspruch 1, wobei R eine verzweigtkettiges C₁₀- bis C₄₀-Alkyl ist.

6. Verfahren nach einem der vorhergehenden Ansprüche, wobei das Alkohol die Formel (Ia) aufweist: 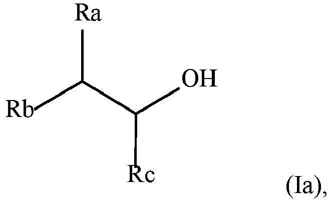 wobei Rₐ und R_{b} unabhängig aus C₃- bis C₁₈-Alkyl, vorzugsweise geradkettigem C₆- bis C₁₈-Alkyl ausgewählt sind und R_{c} aus H und C₁- bis C₄-Alkyl ausgewählt ist.

7. Verfahren nach einem der vorhergehenden Ansprüche, wobei der Alkohol aus 2-Decyl-1-tetradecanol, 2-Octyl-1-dodecanol, 2-Hexyl-1-decanol und Kombinationen davon ausgewählt ist.

8. Verfahren nach einem der vorhergehenden Ansprüche, wobei das Alken die Formel (II) aufweist: 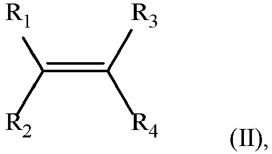 wobei R₁ und R₂ unabhängig aus H, C₁- bis C₆-Alkyl und C₃- bis C₆-Cycloalkyl ausgewählt sind und R₃ und R₄ unabhängig aus C₁- bis C₆-Alkyl und C₃- bis C₆-Cycloalkyl ausgewählt sind.

9. Verfahren nach Anspruch 8, wobei R₁ und R₂ unabhängig aus H und C₁- bis C₄-Alkyl ausgewählt sind, vorzugsweise wobei mindestens eines von R₁ und R₂ C₁- bis C₄-Alkyl ist, und R₃ und R₄ unabhängig aus C₁- bis C₄-Alkyl ausgewählt sind.

10. Verfahren nach Anspruch 9, wobei R₁ und R₂ unabhängig aus H und C₁- bis C₂-Alkyl ausgewählt sind, vorzugsweise wobei mindestens eines von R₁ und R₂ C₁- bis C₂-Alkyl ist, und R₃ und R₄ unabhängig aus C₁- bis C₂-Alkyl ausgewählt sind.

11. Verfahren nach einem der vorhergehenden Ansprüche, wobei das Alken ein C₄- bis C₆-Alken ist.

12. Verfahren nach einem der vorhergehenden Ansprüche, wobei das Alken aus *Iso*Buten, 2,3-Dimethyl-2-buten, 2,3-Dimethyl-1-buten, 2-Methyl-2-buten, 2-Methyl-1-buten und Kombinationen davon ausgewählt ist.

13. Verfahren nach einem der vorhergehenden Ansprüche, wobei der in dem Verfahren gebildete Ether eine Gesamtanzahl von Kohlenstoffatomen von 20 bis 50, vorzugsweise von 22 bis 40, mehr bevorzugt von 24 bis 30 Kohlenstoffatomen und am meisten bevorzugt 28 bis 30 Kohlenstoffatomen aufweist.

14. Verfahren nach einem der vorhergehenden Ansprüche, wobei der Unterschied der Siedepunkte des Alkohols und des aus dem Verfahren erhaltenen Ethers weniger als 50 °C, beispielsweise weniger als 20 °C oder weniger als 10 °C beträgt.

15. Verfahren nach einem der vorhergehenden Ansprüche, wobei das saure Ionenaustauschharz ein saures Ionenaustauschharz vom makroretikulären Typ oder ein saures Ionenaustauschharz vom Gel-Typ ist, vorzugsweise wobei das Harz vom makroretikulären Typ oder Gel-Typ ein starkes Säureharz, wie ein sulfoniertes Harz, ist.

16. Verfahren nach einem der vorhergehenden Ansprüche, wobei das Aluminiumsilicat-Veretherungskatalysator ein Zeolith ist, vorzugsweise wobei der Zeolith mindestens einen Kanal aufweist, der durch einen 10-gliedrigen oder 12-gliedrigen Ring definiert ist.

17. Verfahren nach einem der vorhergehenden Ansprüche, wobei der Aluminiumsilicat-Veretherungskatalysator Zeolith H-Y ist.

18. Verfahren nach einem der vorhergehenden Ansprüche, wobei die Reaktion mit den Reaktanten in der flüssigen Phase betrieben wird.

19. Verfahren nach einem der vorhergehenden Ansprüche, wobei die Reaktanten bei einer Temperatur im Bereich von 30 °C bis 120 °C, vorzugsweise von 50 °C bis 100 °C, mehr bevorzugt von 70 °C bis 90°C, beispielsweise 80 °C, in Kontakt gebracht werden.

20. Verfahren nach einem der vorhergehenden Ansprüche, wobei die Reaktanten bei einem Druck von 100 kPa bis 1000 kPa, vorzugsweise von 100 kPa bis 500 kPa, mehr bevorzugt von 100 kPa bis 250 kPa in Kontakt gebracht werden.

21. Verfahren nach einem der vorhergehenden Ansprüche, wobei das Etherprodukt aus dem Reaktionsgemisch mittels Destillation isoliert wird, vorzugsweise wobei die Destillation bei subatmosphärischem Druck betrieben wird.

22. Verfahren zur Herstellung einer Schmiermittelzusammensetzung, umfassend ein Herstellen eines Ethers gemäß dem Verfahren nach einem der vorhergehenden Ansprüche und ein Mischen des aus dem Verfahren erhaltenen Ethers in eine Schmiermittelzusammensetzung.

23. Verfahren nach Anspruch 22 und in dem Ausmaß seiner Abhängigkeit von einem der Ansprüche 1 bis 20, wobei der erhaltene Ether vor dem Mischen des Ethers in die Schmiermittelzusammensetzung keinem bzw. keinen Zwischenrestalkoholentfernungsschritten unterzogen wird.

24. Verfahren nach Anspruch 22 oder Anspruch 23, wobei der Ether mit einem oder mehreren zusätzlichen Basisstammlösungen und/oder einem oder mehreren Schmiermittel additiven gemischt wird.

25. Verfahren nach einem der Ansprüche 22 bis 24, wobei der Ether in der Schmiermittelzusammensetzung in einer Menge von mehr als 10 Gew.-%, vorzugsweise mehr als 20 Gew.-%, mehr bevorzugt mehr als 30 Gew.-% vorliegt.

26. Verfahren zum Schmieren einer Oberfläche, umfassend ein Herstellen einer Schmiermittelzusammensetzung gemäß dem Verfahren nach einem der Ansprüche 22 bis 25 und ein Zuführen der Schmiermittelzusammensetzung zu einer Oberfläche zum Schmieren, wie einer Oberfläche in einem Verbrennungsmotor.

## Revendications

1. Procédé destiné à préparer un éther, ledit procédé comprenant la mise en contact d'un alcool avec un alcène en présence d'un catalyseur d'éthérification à base de résine échangeuse d'ions acide et/ou d'un catalyseur d'éthérification à base d'aluminosilicate, dans lequel :
i) l'alcool comprend un groupe hydrocarbyle aliphatique en C₈₊ de formule (I) :
R-OH (I)
dans lequel R est un alkyle à chaîne ramifiée de C₈ à C₄₀ ;
ii) l'alcène comprend un atome de carbone oléfinique tertiaire ; et
iii) l'alcène est présent en excès molaire par rapport à l'alcool.

2. Procédé selon la revendication 1, dans lequel le rapport molaire de l'alcène à l'alcool est d'au moins 2/1, de manière préférée d'au moins 4/1, de manière plus préférée d'au moins 6/1, de manière préférée entre toutes d'au moins 8/1.

3. Procédé selon la revendication 1 ou la revendication 2, dans lequel le rapport molaire de l'alcène à l'alcool n'est pas supérieur à 40/1, de manière préférée pas supérieur à 20/1, de manière plus préférée pas supérieur à 16/1, et de manière préférée entre toutes pas supérieur à 12/1.

4. Procédé selon l'une quelconque des revendications 1 à 3, dans lequel le rapport molaire de l'alcène à l'alcool va de 2/1 à 40/1, de manière préférée de 4/1 à 20/1, de manière plus préférée de 6/1 à 16/1, de manière préférée entre toutes de 8/1 à 12/1.

5. Procédé selon la revendication 1, dans lequel R est un alkyle à chaîne ramifiée en C₁₀ à C₄₀.

6. Procédé selon l'une quelconque des revendications précédentes, dans lequel l'alcool est de formule (Ia) : 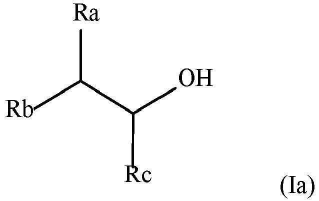 dans lequel, Rₐ et R_{b} sont choisis indépendamment parmi un alkyle en C₃ à C₁₈, de manière préférée un alkyle en C₆ à C₁₈ à chaîne linéaire, et R_{c} est choisi parmi H et un alkyle en C₁ à C₄.

7. Procédé selon l'une quelconque des revendications précédentes, dans lequel l'alcool est choisi parmi un 2-décyl-1-tétradécanol, un 2-octyl-1-dodécanol, un 2-hexyl-1-décanol et des combinaisons de ceux-là.

8. Procédé selon l'une quelconque des revendications précédentes, dans lequel l'alcène est de formule (II) : 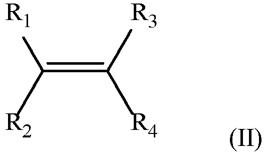 dans lequel, R₁ et R₂ sont choisis indépendamment parmi H, un alkyle en C₁ à C₆ et un cycloalkyle en C₃ à C₆ et R₃ et R₄ sont choisis indépendamment parmi un alkyle en C₁ à C₆ et un cycloalkyle en C₃ à C₆.

9. Procédé selon la revendication 8, dans lequel R₁ et R₂ sont choisis indépendamment parmi H et un alkyle en C₁ à C₄, de manière préférée dans lequel au moins l'un de R₁ et R₂ est un alkyle en C₁ à C₄, et R₃ et R₄ sont choisis indépendamment parmi un alkyle en C₁ en C₄.

10. Procédé selon la revendication 9, dans lequel R₁ et R₂ sont choisis indépendamment parmi H et un alkyle en C₁ à C₂, de manière préférée dans lequel au moins l'un de R₁ et R₂ est un alkyle en C₁ à C₂, et R₃ et R₄ sont choisis indépendamment parmi un alkyle en C₁ à C₂.

11. Procédé selon l'une quelconque des revendications précédentes dans lequel l'alcène est un alcène en C₄ à C₆.

12. Procédé selon l'une quelconque des revendications précédentes, dans lequel l'alcène est choisi parmi un iso-butène, un 2,3-diméthyl-2-butène, un 2,3-diméthyl-1-butène, un 2-méthyl-2-butène, un 2-méthyl-1-butène et des combinaisons de ceux-là.

13. Procédé selon l'une quelconque des revendications précédentes, dans lequel l'éther formé dans le procédé a un nombre total d'atomes de carbone allant de 20 à 50, de manière préférée de 22 à 40, de manière plus préférée de 24 à 30 atomes de carbone, et de manière préférée entre toutes 28 à 30 atomes de carbone.

14. Procédé selon l'une quelconque des revendications précédentes, dans lequel la différence entre les points d'ébullition de l'alcool et de l'éther obtenu à partir du procédé est inférieure à 50 °C, par exemple inférieure à 20 °C ou inférieure à 10 °C.

15. Procédé selon l'une quelconque des revendications précédentes, dans lequel la résine échangeuse d'ions acide est une résine échangeuse d'ions acide de type macroréticulaire ou une résigne échangeuse d'ions acide de type gel, de manière préférée où la résine de type macroréticulaire ou de type gel est une résine acide forte, telle qu'une résine sulfonée.

16. Procédé selon l'une quelconque des revendications précédentes, dans lequel le catalyseur d'éthérification à base d'aluminosilicate est une zéolite, de manière préférée où la zéolite a au moins un canal défini par un noyau de 10 éléments ou de 12 éléments.

17. Procédé selon l'une quelconque des revendications précédentes, dans lequel le catalyseur d'éthérification à base d'aluminosilicate est une zéolite H-Y.

18. Procédé selon l'une quelconque des revendications précédentes, dans lequel la réaction est réalisée avec les réactifs en phase liquide.

19. Procédé selon l'une quelconque des revendications précédentes, dans lequel les réactifs sont mis en contact à une température dans la plage allant de 30 °C à 120 °C, de manière préférée allant de 50 °C à 100 °C, de manière plus préférée allant de 70 °C à 90°C, par exemple 80 °C.

20. Procédé selon l'une quelconque des revendications précédentes, dans lequel les réactifs sont mis en contact à une pression allant de 100 kPa à 1 000 kPa, de manière préférée allant de 100 kPa à 500 kPa, de manière plus préférée allant de 100 kPa à 250 kPa.

21. Procédé selon l'une quelconque des revendications précédentes, dans lequel le produit d'éther est isolé du mélange réactionnel au moyen d'une distillation, de manière préférée où la distillation est réalisée à une pression sub-atmosphérique.

22. Procédé destiné à préparer une composition de lubrifiant, comprenant la préparation d'un éther selon le procédé de l'une quelconque des revendications précédentes, et le mélange de l'éther obtenu à partir du procédé en une composition de lubrifiant.

23. Procédé selon la revendication 22, et dans la mesure où elle dépend de l'une quelconque des revendications 1 à 20, dans lequel l'éther obtenu ne subit aucune étape d'élimination d'alcool résiduel intermédiaire avant le mélange de l'éther en la composition de lubrifiant.

24. Procédé selon la revendication 22 ou la revendication 23, dans lequel l'éther est mélangé avec une ou plusieurs substances mères de base supplémentaires et/ou un ou plusieurs additifs de lubrifiant.

25. Procédé selon l'une quelconque des revendications Claims 22 à 24, dans lequel l'éther est présent dans la composition de lubrifiant en une quantité supérieure à 10 % en poids, de manière préférée supérieure à 20 % en poids, de manière plus préférée supérieure à 30 % en poids.

26. Procédé destiné à lubrifier une surface, comprenant la préparation d'une composition de lubrifiant selon le procédé de l'une quelconque des revendications 22 à 25, et la fourniture de la composition de lubrifiant à une surface pour la lubrification, telle qu'une surface dans un moteur à combustion interne.
